# EUROPÄISCHE PATENTSCHRIFT

(11) **EP 2 455 049 B1**
(45) Veröffentlichungstag und Bekanntmachung des Hinweises auf die Patenterteilung: **20.02.2013**
(21) Anmeldenummer: 10401194.5
(22) Anmeldetag: 12.11.2010
(51) Int. Cl.: A61F 15/00

(54) **Spender**
Dispenser
Distributeur

(43) Veröffentlichungstag der Anmeldung: 23.05.2012
(73) Patentinhaber: Scheffer, Georg, 59846 Sundern (DE)
(72) Erfinder: Scheffer, Georg, 09618 Brand-Erbisdorf (DE)
(74) Vertreter: Steiniger, Carmen

(56) Entgegenhaltungen:
- EP-A2- 0 860 137
- WO-A1-03/060843
- DE-A1- 2 618 007
- DE-U1- 8 205 209
- GB-A- 514 160
- US-A- 2 683 641

## Beschreibung

Die vorliegende Erfindung betrifft einen Spender für rechteckige Tupfer, wobei die Tupfer Abschnitte eines sich in einer Längsrichtung erstreckenden Tupferbandes sind, welches in einer Querrichtung des Tupferbandes Perforationen aufweist, an welchen die Tupfer von dem Tupferband abtrennbar sind, wobei ein Ende des Tupferbandes durch wenigstens eine Entnahmeöffnung des Spenders führbar ist und in einer Entnahmerichtung aus dem Spender ziehbar Ist, wobei die Entnahmerichtung gleich der Längsrichtung des Tupferbandes ist.

Spender der genannten Gattung sind im Stand der Technik bekannt. Gebräuchliche Spender sind beispielsweise Pappkartons, die eine Rolle von Tupferband beherbergen und an einer Kante eine schlitzförmige Öffnung aufweisen, durch welche das Tupferband herausgezogen werden kann. Von dem Tupferband können Tupfer abgerissen werden. Die Tupfer werden dann für kosmetische, medizinische oder sonstige Zwecke eingesetzt. Die Tupfer selbst sind welche Flächengeblide, beispielsweise aus Zellstoff bestehend. Nachteilig an den bekannten Spendern ist, dass das Tupferband an der Entnahmeöffnung der Tupferverpackung nicht gehalten wird und in die Verpackung hineinrutschen kann. Ein weiterer Nachteil besteht darin, dass zum Abreißen von Tupfern zwei Hände benötigt werden, wobei eine Hand zum Halten des Tupferstreifens und die zweite Hand zum Anfassen und Abreißen des Tupfers benötigt wird. Eine solche Zweihandbedlenung des Spenders ist ungünstig, wenn dem Benutzer des Spenders nur eine Hand zum Entnehmen der Tupfer zur Verfügung steht, beispielsweise weil die andere Hand mit dem Halten eines anderen Gegenstandes beschäftigt ist.

Die Druckschrift WO 03/060843 A1 beinhaltet Spender für Dilatoren aus Tissue-Material. Die Dilatoren dienen zum Aufweiten von Nasenhöhlen, um temporär die Atmung verbessern zu können. Der Spender soll dazu dienen, dem Nutzer einen Dilator möglichst rasch und leicht zur Verfügung stellen zu können und die Dilatoren vor Beschädigung zu schützen. Die Dilatoren können beispielsweise in Taschen, die in einer Rolle zusammengefasst sind, zur Verfügung gestellt werden. Die Taschen sind durch Kaltsiegelung von zwei Materiallagen ausgebildet. Wenn ein Dilator aus einer Entnahmeöffnung des Spenders herausgeführt wird, sind die Materiallagen, zwischen welchen sich der Dilator befindet, bereits voneinander getrennt, sodass der dazwischen befindliche Dilator einfach entnommen werden kann. Beidseitig der Entnahmeöffnung sind feine Abreißzähne vorgesehen, an welchen das sich dort ansammelnde Verpackungsmaterial für die Dilatoren abgerissen werden kann.

Die Druckschrift DE 2,683,641 A offenbart einen Papierrollenspender zum Abreißen von Abschnitten einer Papierrolle. Die Papierrolle weist keine Perforationen auf. An dem Spender ist eine Abreißplatte vorgesehen, an deren Ende eine Kante mit Abreißzähnen ausgebildet ist. Die Kante mit den Abreißzähnen ist nach unten in Richtung der abzureißenden Papierrolle ausgerichtet Das Papier muss somit zum Abreißen nach oben gezogen werden.

In der Druckschrift GB 514,160 A ist ein Spender für Krepppapier in Rollenform beschrieben. Solches Krepppapier weist keine Perforationen auf. Die Abtrennung von Papierabschnitten von der Rolle erfolgt mittels eines Abreißkanals, dessen gebogenem Ende Abreißzähne gegenüber angeordnet sind.

Die Druckschrift DE 26 18 007 A schlägt offene, trogförmige Papierrollenhalter, beispielsweise zur Aufnahme von Toilettenpapier, vor. Die Tröge dieser Papierrollenhalter weisen jeweils an einer Stirnseite eine Abrisskante auf, die entweder mit Abreißzähnen oder einem Werkstoff mit hohem Reibwert, wie beispielsweise Moosgummi, versehen ist. Die Zähne können verschiedene Formen aufweisen.

Die Druckschrift DE 82 05 209 U1 beschreibt einen Spender für Kunststoff-Folienbeutel. Dieser Spender weist eine Zunge auf, die für ein Hindurchgleiten durch einen Teil-Durchschnitt in der Abreißperforation einer Schlauchfolie vorgesehen ist. Die Zunge hält beim Abreißen eines Beutels von der Schlauchfolie diese fest, sodass ein vorderer, noch an der Schlauchfolie hängender Beutel mit relativ geringem Kraftaufwand entlang der Abreißperforation, in welcher die Zunge liegt, abgerissen werden kann. Die Zunge ist an eine Wand des Spenders geklemmt und dabei stufenlos höhenverstellbar. Alternativ können auch zwei Zungen an die Spenderwand geklemmt werden. Der Spender dieser Druckschrift ist in Hülsenform ausgebildet, wobei In der Hülse ein vertikal nach unten durchgehender Entnahmespalt für die Folienbeutel vorgesehen ist. Dabei befindet sich die höhenverstellbare Zunge direkt an einer Seitenkante der Hülse und ragt in Richtung des Entnahmespaltes.

Die Druckschrift EP 0 860 137 A2 offenbart einen Spender, beispielsweise für Toilettenpapierrollen, der eine Öffnung aufweist, die schmaler als die Breite des im Spender befindlichen Papierbandes ist. Zum Abreißen des Papiers kann dieses wahlweise durch die Öffnung oder über eine Abreißkante gerührt werden.

Es ist daher die Aufgabe der vorliegenden Erfindung, einen gattungsgemäßen Spender bereitzustellen, der kostengünstig realisierbar ist und der eine komfortable Entnahme von Tupfern ermöglicht.

Die Aufgabe wird dadurch gelöst, dass an der Entnahmeöffnung und/oder der Entnahmeöffnung in der Entnahmerichtung nachgeordnet wenigstens eine Auskragung für einem Durchgriff durch wenigstens eine der Perforationen des Tupferbandes vorgesehen ist.

Die Auskragung ist dabei ein mechanisches Objekt, das an wenigstens einer Perforation durch das Tupferband hindurchgreift. Die Auskragung ist also im Wesentlichen in Richtung der Oberflächennormale des Tupferbandes ausgerichtet, wobei die Ausrichtung nicht genau senkrecht zu dem Tupferband ausgerichtet sein muss. Wichtig ist nur, dass die Auskragung dem Tupferband bei Zug des Tupferbandes in der Entnahmerichtung einen Widerstand entgegensetzt, sodass das Tupferband von der Auskragung zurückgehalten wird, während ein Tupfer von dem Tupferband abgerissen wird. Die Form der Auskragung ist so gestaltet, dass ein Durchdringen des Tupferbandes an dessen Perforation(en) behinderungsfrei möglich ist. Der Bereich der Auskragung, der das Tupferband durchdringt, hat also eine geringere Breite als die wenigstens eine durch Perforation hergestellte Öffnung in dem Tupferband, durch die die Auskragung durchgeführt wird.

Beispielsweise kann die Auskragung ein Winkel sein. Der Winkel kann dabei so von dem Spender auskragen, dass sein abgewinkeltes Ende, welches für den Durchgriff durch wenigstens eine der Perforationen des Tupferbandes vorgesehen ist, im Hinblick auf eine Fläche, auf welcher der Spender steht, nach oben ausgerichtet ist. Es ist jedoch auch möglich, dass das winklige Ende des Winkels im Hinblick auf die Oberfläche, auf welcher der Spender steht, nach unten oder zur Seite ausgerichtet ist, wenn beispielsweise das Tupferband nach oben abgezogen werden soll oder senkrecht aus dem Spender führt.

Es ist besonders günstig, wenn die Auskragung aus Metall, wie beispielsweise aus Aluminium, ausgebildet ist. Aus dem Metall kann auf relativ einfache Weise ein Winkel oder eine andere geeignete Auskragung geformt werden. Darüber hinaus ist die Auskragung, wenn sie aus Metall ausgebildet ist, sehr stabil und sichert somit ein dauerhaft günstiges Abreiβen der Tupfer vom Tupferband.

In einer bevorzugten Ausbildung des erfindungsgemäßen Spenders hat die Auskragung eine flache, an der dem Tupferband zugewandten Kante halbrunde Form eines Biberschwanzdachziegels. Diese Auskragung hat eine abgerundete Kante, über die ein Gleiten des Tupferbandes vorgesehen ist. Durch die runde Kante wird eine geringe Reibung zwischen Tupferband und Auskragung erreicht, wodurch ein Eindringen der Auskragung in die Oberfläche des Tupfers verhindert wird. Durch die relativ große Breite des Flachprofils wird die Rückhaltekraft für das Tupferband auf eine entsprechend große Kante verteilt. Dadurch wird eine Deformation des Tupferbandes in Folge der Rückhaltekraft vermieden, und das Halten des Tupferbandes in der Entnahmerichtung wird unterstützt.

Statt eines biberschwanzförmigen Flachprofils kann für die Auskragung jedoch auch ein beliebiges anderes Profil, beispielsweise ein stabförmiges Profil, verwendet werden. Es können auch mehrere Auskragungen nebeneinander vorgesehen sein, die in eine oder mehrere Öffnungen oder Schlitze der Perforation eingreifen. In anderen Ausführungsformen der vorliegenden Erfindung können jedoch auch andere Formen der Auskragung, wie beispielsweise eine Dreieckform oder eine rechteckige Form verwendet werden.

Der erfindungsgemäße Spender eignet sich für Tupfer, die aus mehreren Lagen ausgebildet sind, wobei die Außenlagen der Tupfer aus Zellstoff-Tissue und wenigstens eine Innenlage der Tupfer aus Zellstoffwatte ausgebildet ist. Dabei ist die in der Mitte der Tupfer befindliche Zellstoffwatte beidseitig mit dem Zellstoff-Tissue-Material der Außenlagen verquetscht. Diese Tupfer zeichnen sich durch eine besonders vorteilhafte Saugfähigkeit aus, können einfach perforiert werden und sind damit mit dem erfindungsgemäßen Spender leicht voneinander separierbar.

Besonders vorteilhaft kann der erfindungsgemäße Spender für das Abreißen von Tupfern von einem zweiseitigen Tupferband verwendet werden, welches jeweils zwei nebeneinander angeordnete Tupfer aufweist. Bei dieser Ausführungsform des erfindungsgemäßen Spenders sind an dem Spender zwei Auskragungen für einen Durchgriff durch zwei Perforationen vorgesehen, die zwischen zwei nebeneinander angeordneten Tupfern und zwei weiteren in Entnahmerichtung nachfolgenden Tupfern vorgesehen sind.

In einer vorteilhaften Ausgestaltung der Erfindung ist der Spender als Spendeständer zur Aufnahme einer Tupferverpackung ausgebildet, wobei der Spendeständer die Auskragung aufweist. Der Spendeständer ist eine wiederverwendbare Vorrichtung, in die nach Verbrauch einer Tupferverpackung erneut eine Tupferverpackung eingestellt werden kann. Der Spendeständer unterliegt keinem Verschleiß und kann beliebig oft wiederverwendet werden. Entsprechend der großen Lebensdauer des Spendeständers sind moderat hohe Herstellungskosten akzeptabel und der Tupferständer kann entsprechend formschön und stabil ausgebildet werden. Der Spendeständer und die daran befestigte Auskragung können aus beliebigen Materialien, beispielsweise aus Kunststoff und/oder Metall, hergestellt sein. Der Spendeständer kann zumindest teilweise transparent sein oder einen Aufdruck, beispielsweise einen Werbeaufdruck, tragen. Der Spendeständer kann aber auch beliebig farblich gestaltet sein.

In einem günstigen Ausführungsbeispiel des erfindungsgemäßen Spenders ist an dem Spendeständer die Position der Auskragung an die Position der Entnahmeöffnung der Tupferverpackung anpassbar. Die Tupferverpackungen sind nicht standardisiert und es gibt verschiedene Tupferverpackungen, bei denen die Entnahmeöffnung(en) an unterschiedlichen Stellen vorgesehen ist bzw. sind. Durch die Verstellbarkeit des Spendeständers kann dieser an verschiedene Tupferverpackungen angepasst werden, sodass der Spendeständer für verschiedene Tupferverpackungen verwendbar ist.

In einer alternativen Ausbildung der Erfindung ist der Spender als Spendeverpackung ausgebildet, wobei die Spendeverpackung die Auskragung aufweist. In dieser Variante der Erfindung wird die Verpackung für das Tupferband so ausgebildet, dass sie die Funktion des erfindungsgemäßen Spenders erfüllt. Zu diesem Zweck wird die Auskragung als Bestandteil der Spendeverpackung realisiert. Dabei kann die Auskragung aus dem Hauptmaterial der Verpackung ausgebildet sein. Beispielsweise kann eine geeignete Form in eine Pappverpackung gestanzt sein, um hierdurch die Auskragung auszubilden. Die Auskragung kann jedoch auch als separates Bauteil der Verpackung beigelegt sein und an dieser anbringbar sein. Mit der Spendeverpackung können die Vorteile der Erfindung mit geringstem Aufwand umgesetzt werden.

Der Spender weist eine Führung für das Tupferband auf, wobei die Führung in der Entnahmerichtung des Tupferbandes zwischen der Entnahmeöffnung und der Auskragung vorgesehen ist. Durch die Führung wird ein Zurückgleiten des Endes des Tupferbandes in die Tupferverpackung vermieden und das Ende des Tupferbandes wird an der benötigten Position des Spenders bereitgehalten. Das Bereithalten des Tupferbandes stellt einen wesentlichen Komfortgewinn bei dem erfindungsgemäßen Spender dar. Die Führung kann sowohl in einem Spendeständer als auch in einer Spendeverpackung ausgebildet sein. Durch die Führung wird erreicht, dass das Ende des Tupferbandes ausreichend weit von der Entnahmeöffnung der Tupferverpackung entfernt ist, um ein Zurückgleiten des Endes des Tupferbandes in die Tupferverpackung zu verhindern.

In einer besonders bevorzugten Ausführungsform des erfindungsgemäßen Spenders ist die Auskragung von der Führung beabstandet. Zwischen dem Ende der Führung und der Auskragung liegt das Tupferband zumindest teilweise frei in einer definierten Position. In dieser Position ist ein komfortables Ergreifen des Tupferbandes und ein Führen des Tupferbandes über die Auskragung zum Zwecke der Abtrennung eines Tupfers möglich.

Es hat sich als besonders vorteilhaft erwiesen, den erfindungsgemäßen Spender so auszubilden, dass an der Seite des Spenders, an der die Entnahmeöffnung vorgesehen ist, ein längerer Fuß des Spenders als an der gegenüberliegenden Seite vorgesehen ist, sodass der Spender verkippt bzw. in einem Winkel aufstellbar ist. Hierdurch kann eine erhöhte Standsicherheit des Spenders zur Verfügung gestellt werden, dessen Gleichgewicht sich durch die verkippte Aufstellung in Richtung des Tupferbandes verlagert. Hierdurch ist ein komfortableres Abreißen der Tupfer möglich.

Es ist besonders günstig, wenn der Fuß höhenverstellbar ist. Hierdurch kann durch den Anwender des Spenders die Standsicherheit definiert und der Spender an die jeweiligen Gegebenheiten angepasst werden.

In einem weiteren Ausführungsbeispiel der vorliegenden Erfindung wird der Fuß seitlich in Gleitführungen gehalten. Innerhalb dieser Gleitführungen kann der Fuß so verschoben werden, dass seine Länge relativ zu der Oberfläche, auf welcher der Spender aufgestellt wird, verstellt werden kann.

In einer weiteren Ausführungsform der vorliegenden Erfindung ist die Höhe der Verkippung mittels wenigstens einer an dem Fuß vorgesehenen Arretiervorrichtung feststellbar. Hierdurch kann der Anwender des Spenders wahlweise eine geeignete Verkippung des Spenders relativ zu der Oberfläche, auf welcher er steht, einstellen.

### Bevorzugte Ausführungsformen der vorliegenden Erfindung, deren Aufbau, Funktion und Vorteile sollen im Folgenden anhand von Figuren näher erläutert werden, wobei

- Figur 1: schematisch einen Abschnitt eines Tupferbandes in einer Draufsicht zeigt;
- Figur 2: schematisch einen Abschnitt eines zweireihigen Tupferbandes in einer Draufsicht zeigt;
- Figur 3: schematisch eine Ausführungsform eines erfindungsgemäßen Spenders mit einem Spendeständer in einer Seitenansicht zeigt;
- Figur 4: schematisch eine Seitenansicht einer Spendeverpackung zeigt;
- Figur 5: schematisch eine alternative Ausführungsform eines erfindungsgemäßen Spenders in Form einer Spendeverpackung zeigt;
- Figur 6: schematisch eine weitere alternative Ausführungsform eines erfindungsgemäßen Spenders in Form einer Spendeverpackung zeigt; und
- Figur 7: schematisch noch eine weitere Ausführungsform eines erfindungsgemäβen Spenders mit verkippter Aufstellung zeigt.

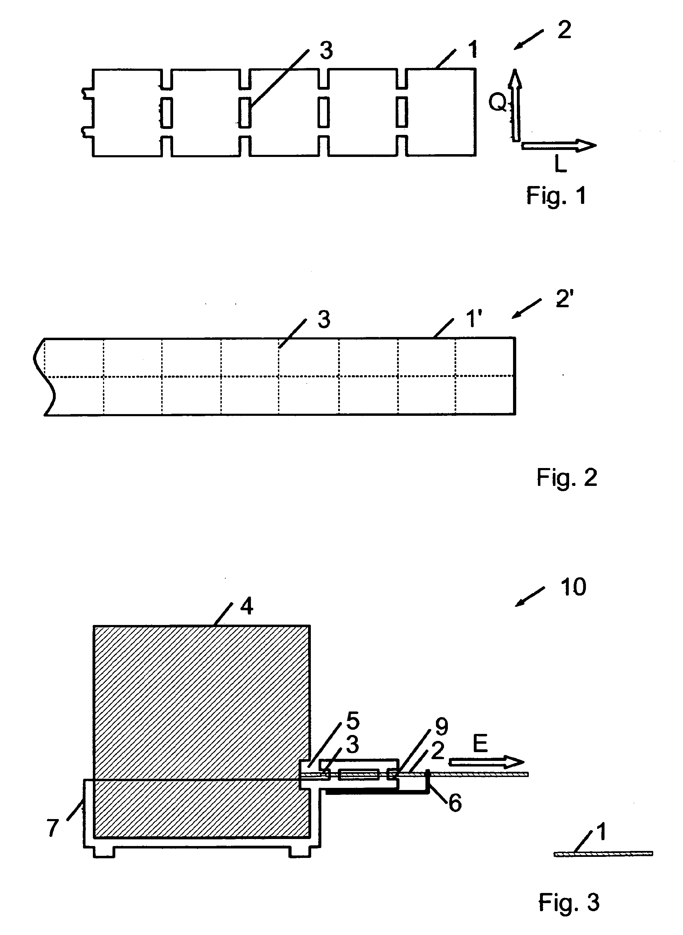

Figur 1 zeigt schematisch einen Abschnitt eines Tupferbandes 2, für das der erfindungsgemäße Spender 10, 11, 12, 14 konzipiert ist. Das Tupferband 2 hat eine Längsrichtung L und eine Querrichtung Q. In der dritten, nicht dargestellten Raumrichtung weist das Tupferband 2 eine geringe Dicke auf. Das Tupferband 2 ist durch Perforationen 3 in Tupfer 1 unterteilt. Die Perforationen 3 sind vorzugsweise langgestreckte Schlitze, die in dem Material des Tupferbandes 2 ausgebildet sind und von schmalen Stegen unterbrochen sind, wobei die Stege die einzelnen Tupfer 1 zusammenhalten. In Figur 1 sind die Perforationen 3 zur Veranschaulichung überdimensioniert dargestellt. In diesem Ausführungsbeispiel werden benachbarte Tupfer 1 nur durch zwei Stege zusammengehalten. In anderen, nicht dargestellten Ausführungen können auch beliebige andere Perforationsmuster eingesetzt werden.

In Figur 2 ist schematisch ein Abschnitt eines Tupferbandes 2' dargestellt. Dieses Tupferband 2' enthält in zwei Reihen nebeneinander angeordnete Tupfer 1'. Das Tupferband 2' weist sowohl in Querrichtung Q als auch in Längsrichtung L Perforationen 3 auf.

Die Tupfer 1 bzw. 1' sind vorzugsweise sogenannte Profipad-Tupfer. Die Profipad-Tupfer sind mehrlagig ausgebildet, wobei in der Mitte der Tupfer Zellstoffwatte vorgesehen ist, welche beidseitig mit Zellstoff-Tissue verquetscht ist. Der erfindungsgemäße Spender 10, 11, 12, 14 eignet sich jedoch auch zum Entnehmen bzw. Abreißen anderer Tupfer.

Figur 3 zeigt schematisch eine mögliche Ausführungsform eines erfindungsgemäßen Spenders 10. Der Spender 10 weist einen Spendeständer 7 auf, der zur Aufnahme einer Tupferverpackung 4 dient. Die Tupferverpackung 4 dient in dem gezeigten Ausführungsbeispiel zur Verpackung eines spiralförmig gewickelten Tupferbandes 2. In anderen, nicht dargestellten Ausbildungen der Erfindung können auch Tupferverpackungen 4 für gefaltete Tupferbänder 2 und verpackte Tupferbänder 2 oder anderweitig bereitgestellte Tupferbänder 2 zum Einsatz kommen.

Zur Entnahme des Tupferbandes 2 aus der Tupferverpackung 4 ist eine Entnahmeöffnung 5 in der Tupferverpackung 4 vorgesehen. Durch die Entnahmeöffnung 5 wird das Tupferband 2 in einer Entnahmerichtung E entnommen. In dem dargestellten Ausführungsbeispiel weist der Spendeständer 7 eine Führung 9 auf, durch die das Tupferband 2 geführt wird. Die Führung 9 ist in dem dargestellten Beispiel aus zwei gegenüber befindlichen flachen Bereichen ausgebildet, zwischen welchen ein Schlitz für die Durchführung des Tupferbandes 2 vorgesehen ist.

Der Spendeständer 7 weist ferner eine Auskragung 6 auf, die zum Durchgriff durch wenigstens eine Perforation 3 konzipiert ist. In dem dargestellten Ausführungsbeispiel ist die Auskragung 6 ein Ende eines abgewinkelten Metallblechstreifens, wobei der Winkel als separates Element ausgebildet ist, welcher an einer Unterseite der Führung 9 befestigt ist und dessen abgewinkeltes, hier nach oben ragendes Ende in einem Abstand zu dem Ende der Führung 9 vorgesehen ist. In anderen, nicht dargestellten Ausführungsformen der vorliegenden Erfindung kann die Auskragung 6 auch einstückig mit der Führung 9 oder dann, wenn keine Führung 9 vorgesehen ist, direkt im Anschluss an die Entnahmeöffnung 5 vorgesehen sein.

Der Bereich der Auskragung 6, der in Figur 3 winklig zu der Entnahmerichtung E dargestellt ist, weist in einer Ansicht quer zu der dargestellten Ansicht die Form eines Biberschwanzdachziegels auf. Das heißt, die in der Darstellung obere Kante der Auskragung 6 weist eine halbrunde Form auf, auf der das Tupferband 2 gut gleitet und durch die die Perforation 3 sicher durchdrungen wird. Wenn sich die Auskragung 6, wie hier dargestellt, in einer Perforation 3 befindet, kann ein Tupfer 1 von dem Tupferband 2 abgetrennt werden, indem das Tupferband 2 in die Richtung der Entnahmerichtung E und/oder nach unten gezogen wird.

In anderen, nicht gezeigten Ausführungsvarianten der vorliegenden Erfindung kann die Auskragung 6 bzw. das Ende der Auskragung 6 auch eine andere geeignete Form, wie beispielsweise eine Dreieckform oder eine rechteckige Form aufweisen. Die Auskragung 6 kann auch aus Einzelelementen zusammengesetzt sein. Die Auskragung 6 unterscheidet sich jedoch deutlich von den im Stand der Technik zum Abreißen von Tupfern teilweise verwendeten Abreißzähnen, welche nicht geeignet sind, um einen Durchgriff durch das Tupfermaterial zu gewährleisten. Dagegen ist die erfindungsgemäß vorgeschlagene Auskragung 6 so groß, dass sie die in dem Tupferband 2 vorgesehenen Perforationen 3 durchgreifen kann.

Der Spendeständer 7 ist vorzugsweise so ausgebildet, dass ein Abreißen der Tupfer von dem Tupferband 2 mit einer Hand möglich ist. Die erforderliche Standfestigkeit des Spenders 10 wird durch eine ausreichend große Masse des Spendeständers 7 erreicht.

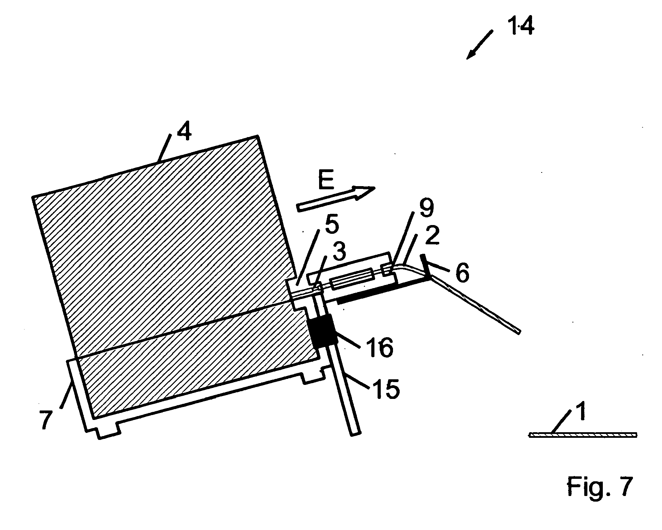

In anderen, beispielsweise in Figur 7 dargestellten Ausbildungen eines erfindungsgemäß einsetzbaren Spendeständers 7, kann dieser in einem Winkel aufgestellt sein, sodass die Auskragung 6 angehoben wird und das Tupferband 2 in einem zum Untergrund des Spendeständers 7 ausgerichteten Winkel abgerissen werden kann. Dadurch wird ein Teil der Abrisskraft vom Untergrund aufgenommen und die Standfestigkeit des Spenders 10 wird erhöht.

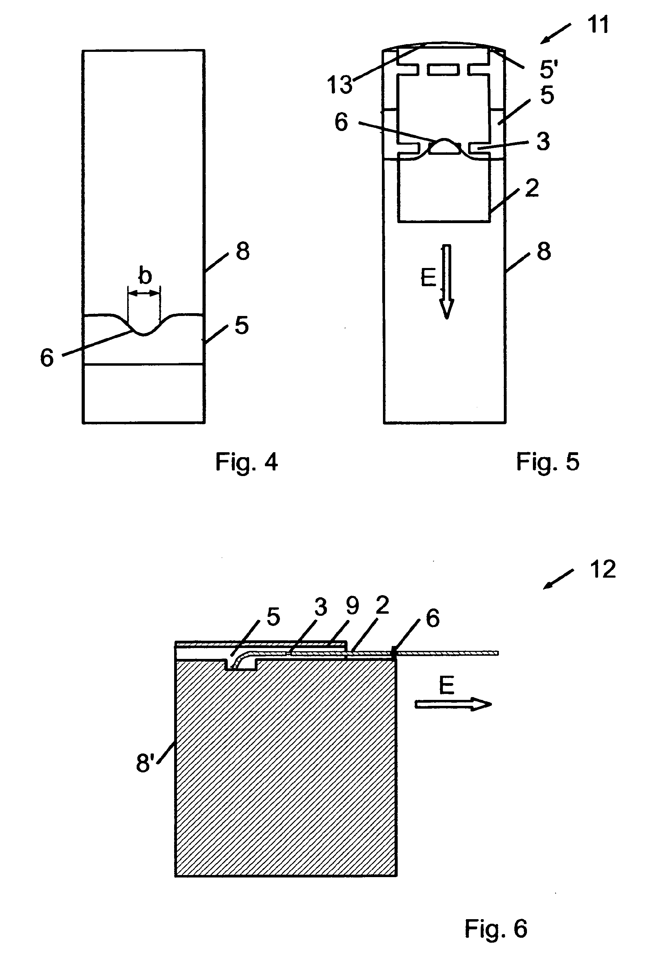

In Figur 4 ist eine Ansicht einer Tupferverpackung dargestellt, die als Spendeverpackung 8 ausgebildet ist. Die Spendeverpackung 8 weist eine Entnahmeöffnung 5 auf, durch welche nach ihrer Öffnung ein Tupferband 2 entnommen werden kann. In der dargestellten Orientierung kann die Spendeverpackung 8 in einen Spendeständer 7 eingestellt werden und dort als Spender 10 benutzt werden. Für diese Einsatzmöglichkeit könnte die Spendeöffnung 5 auch gerade Kanten besitzen. In dem dargestellten Ausführungsbeispiel weist die Entnahmeöffnung 5 an einer Kante aber eine Nase auf, die als Auskragung 6 verwendet werden kann. Die Auskragung 6 weist eine Breite B auf, die dazu geeignet ist, in die Perforation 3 eines Tupferbandes 2 einzugreifen.

In Figur 5 ist die Verwendung der Spendeverpackung 8 als Spender 11 dargestellt. Das Tupferband 2 wird hier über eine Entnahmeöffnung 5' aus der Spendeverpackung 8 entnommen. Die Entnahmeöffnung 5 wird hier nicht zur Entnahme des Tupferbandes 2, sondern zur Freilegung der Auskragung 6 benötigt. Die Auskragung 6 wird nach Öffnen der Entnahmeöffnung 5 in Richtung Tupferband 2 gebogen, sodass die Auskragung 6 in die Perforation 3 des Tupferbandes 2 eingreifen kann. Durch das Biegen der Auskragung 6 wird diese quer zu der Entnahmerichtung E ausgerichtet und kann dem Tupferband 2 eine Kraft entgegensetzen, die ein Abreißen von Tupfern 1 ermöglicht. An der Entnahmeöffnung 5' wird das Tupferband 2 durch den Verschluss 13 der Spendeverpackung 8 eingeklemmt, sodass das Tupferband 2 nicht selbstständig in die Spendeverpackung 8 zurückfällt.

Figur 6 zeigt schematisch eine Ansicht eines weiteren alternativen erfindungsgemäßen Spenders 12, der eine Spendeverpackung 8' aufweist. In der Spendeverpackung 8' ist eine Führung 9 ausgebildet, durch die das Tupferband 2 zwischen Entnahmeöffnung 5 und Auskragung 6 geführt ist. In diesem Ausführungsbeispiel der Erfindung ist die Auskragung 6 an einer Kante der Spendeverpackung 8' angeordnet, wodurch ein Abreißen der Tupfer 1 in einer von der Entnahmerichtung E abweichenden Richtung vereinfacht wird.

Figur 7 zeigt schematisch eine weitere mögliche Ausführungsvariante eines erfindungsgemäßen Spenders 14. Dabei ist der Spender 14 grundsätzlich ähnlich wie der Spender 10 aus Figur 3 aufgebaut, weist jedoch an seiner Seite, an welcher sich die Entnahmeöffnung 5 befindet, einen längeren Fuß 15 als an der gegenüberliegenden Seite auf. In dem dargestellten Ausführungsbeispiel ist der Fuß 15 höhenverstellbar. Die Höhenverstellbarkeit des Fußes 15 wird in dem dargestellten Ausführungsbeispiel durch beidseitig des Fußes 15 vorgesehene Gleitführungen 16 gewährleistet, in welchen der Fuß 15 nach oben und unten verfahrbar ist. Vorzugsweise kann der Fuß 15 auch in einer oder mehreren Höhenstellungen durch wenigstens eine Arretiervorrichtung arretierbar sein. Durch den längeren Fuß 15 ist eine verkippte Aufstellung des Spenders 14 gegenüber der Oberfläche, auf welcher der Spender 14 abgestellt ist, möglich. Dadurch verlagert sich das Gewicht des Spenders 14 in Richtung des abzurollenden Tupferbandes 2. Dies hat zur Folge, dass der Spender 14 besonders standsicher aufgestellt werden kann.

## Patentansprüche

1. Spender (10, 11, 12, 14) mit einem sich in einer Längsrichtung (L) erstreckenden Tupferband (2) mit rechteckigen Tupfern (1, 1'), die Abschnitte des Tupferbandes (2) sind, wobei das Tupferband (2) in einer Querrichtung (Q) des Tupferbandes (2) Perforationen (3) aufweist, an welchen die Tupfer (1, 1') von dem Tupferband (2) abtrennbar sind, wobei ein Ende des Tupferbandes (2) durch wenigstens eine Entnahmeöffnung (5, 5') des Spenders (10, 11, 12, 14) führbar ist und in einer Entnahmerichtung (E) aus dem Spender (10, 11, 12, 14) ziehbar ist, und wobei die Entnahmerichtung (E) gleich der Längsrichtung (L) des Tupferbandes (2) ist,
**dadurch gekennzeichnet,**
**dass** an der Entnahmeöffnung (5, 5') und/oder der Entnahmeöffnung (5, 5') in der Entnahmerichtung (E) nachgeordnet wenigstens eine Auskragung (6) für einen Durchgriff durch wenigstens eine der Perforationen (3) des Tupferbandes (2) vorgesehen ist, wobei die Perforationen (3) langgestreckte Schlitze sind, die in dem Material des Tupferbandes (2) ausgebildet sind und von schmalen Stegen unterbrochen sind, wobei die Stege die einzelnen Tupfer (1) zusammenhalten, und wobei die Auskragung (6) durch wenigstens eine durch Perforation hergestellte Öffnung in dem Tupferband (2) durchgeführt wird und wobei die Auskragung (6) sich deutlich von zum Abreißen von Tupfern verwendeten Abreißzähnen unterscheidet und dagegen so groß ist, dass sie die in dem Tupferband (2) vorgesehenen Perforationen (3) durchgreifen kann, sodass dann, wenn sich die Auskragung (6) in einer Perforation (3) befindet, ein Tupfer (1, 1') von dem Tupferband (2) abgetrennt werden kann, indem das Tupferband (2) in die Richtung der Entnahmerichtung (E) und/oder nach unten gezogen wird, wobei die Auskragung (6) also im Wesentlichen in Richtung der Oberflächennormale des Tupferbandes (2) ausgerichtet ist und die Auskragung (6) dem Tupferband (2) bei Zug des Tupferbandes (2) in der Entnahmerichtung (E) einen Widerstand entgegensetzt, sodass das Tupferband (2) von der Auskragung (6) zurückgehalten wird, während ein Tupfer (1, 1') von dem Tupferband (2) abgerissen wird, und wobei der Spender (10, 12) eine Führung (9) für das Tupferband (2) aufweist, wobei die Führung (9) in der Entnahmerichtung (E) des Tupferbandes (2) zwischen der Entnahmeöffnung (5, 5') und der Auskragung (6) vorgesehen ist.

2. Spender nach Anspruch 1, **dadurch gekennzeichnet, dass** die Auskragung (6) ein Winkel ist.

3. Spender nach Anspruch 1 oder 2, **dadurch gekennzeichnet, dass** die Auskragung (6) aus Metall ausgebildet ist.

4. Spender nach wenigstens einem der vorhergehenden Ansprüche, **dadurch gekennzeichnet, dass** die Auskragung (6) ein Flachprofil in Form eines Biberschwanzdachziegels ist, wobei die dem Tupferband (2) zugewandte Kante der Auskragung (6) halbrund ausgebildet ist.

5. Spender nach wenigstens einem der vorhergehenden Ansprüche, **dadurch gekennzeichnet, dass** die Tupfer (1 1') aus mehreren Lagen ausgebildet sind, wobei die Außenlage aus Zellstoff-Tissue und wenigstens eine Innenlage der Tupfer (1,1') aus Zellstoffwatte ausgebildet ist.

6. Spender nach Anspruch 5, **dadurch gekennzeichnet, dass** an dem Spender (10, 11, 12, 14) zwei Auskragungen (6) für einen Durchgriff durch zwei Perforationen (3) vorgesehen sind, die zwischen zwei nebeneinander angeordneten Tupfern (1, 1') und zwei weiteren in Entnahmerichtung (E) nachfolgenden Tupfern (1, 1') vorgesehen sind.

7. Spender nach wenigstens einem der vorhergehenden Ansprüche, **dadurch gekennzeichnet, dass** der Spender (10) als Spendeständer (7) zur Aufnahme einer Tupferverpackung (4) ausgebildet ist, wobei der Spendeständer (7) die Auskragung (6) aufweist.

8. Spender nach Anspruch 7, **dadurch gekennzeichnet, dass** an dem Spendeständer (7) die Position der Auskragung (6) an die Position der Entnahmeöffnung (5, 5') der Tupferverpackung (4) anpassbar ist.

9. Spender nach wenigstens einem der Ansprüche 1 bis 6, **dadurch gekennzeichnet, dass** der Spender (11, 12) als Spendeverpackung (8, 8') ausgebildet ist, wobei die Spendeverpackung (8, 8') die Auskragung (6) aufweist.

10. Spender nach wenigstens einem der vorhergehenden Ansprüche, **dadurch gekennzeichnet, dass** die Auskragung (6) von der Führung (9) beabstandet ist.

11. Spender nach wenigstens einem der vorhergehenden Ansprüche, **dadurch gekennzeichnet, dass** der Spender (14) an der Seite, an der die Entnahmeöffnung (5, 5') vorgesehen ist, einen längeren Fuß (15) als an der gegenüberliegenden Seite aufweist, sodass der Spender (14) verkippt aufstellbar ist.

12. Spender nach Anspruch 11, **dadurch gekennzeichnet, dass** der Fuß (15) höhenverstellbar ist.

13. Spender nach Anspruch 12, **dadurch gekennzeichnet, dass** der Fuß (15) seitlich in Gleitführungen (16) gehalten ist.

14. Spender nach Anspruch 11, 12 oder 13 **dadurch gekennzeichnet, dass** die Höhe der Verkippung mittels wenigstens einer an dem Fuß (15) vorgesehenen Arretiervorrichtung feststellbar ist.

## Claims

1. Dispenser (10, 11, 12, 14) having a swab strip (2) which extends in a longitudinal direction (L) and has rectangular swabs (1, 1') which constitute portions of the swab strip (2), wherein the swab strip (2), as seen in a transverse direction (Q) of the swab strip (2), has perforations (3) at which the swabs (1, 1') can be separated from the swab strip (2), wherein one end of the swab strip (2) can be guided through at least one removal opening (5, 5') of the dispenser (10, 11, 12, 14) and can be pulled out of the dispenser (10, 11, 12, 14) in a removal direction (E), and wherein the removal direction (E) is equal to the longitudinal direction (L) of the swab strip (2),
**characterized in that**
at least one projection (6) for engagement through at least one of the perforations (3) of the swab strip (2) is provided at the removal opening (5, 5') and/or downstream of the removal opening (5, 5'), as seen in the removal direction (E), wherein the perforations (3) are elongate slits which are formed in the material of the swab strip (2) and are interrupted by narrow crosspieces, wherein the crosspieces hold the individual swabs (1) together, and wherein the projection (6) is guided through at least one perforated opening in the swab strip (2), and wherein the projection (6) clearly differs from tear-off teeth used for tearing off swabs and, in contrast, is large enough to be able to engage through the perforations (3) provided in the swab strip (2), and therefore, when the projection (6) is located in a perforation (3), a swab (1, 1') can be severed from the swab strip (2) by the swab strip (2) being pulled in the removal direction (E) and/or downwards, wherein the projection (6) is thus oriented essentially in the direction of the surface normal of the swab strip (2) and the projection (6) counters the swab strip (2) with a resistance when the swab strip (2) is pulled in the removal direction (E), and therefore the swab strip (2) is restrained by the projection (6) while a swab (1, 1') is torn off from the swab strip (2), and wherein the dispenser (10, 12) has a guide (9) for the swab strip (2), wherein the guide (9) is provided between the removal opening (5, 5') and the projection (6), as seen in the removal direction (E) of the swab strip (2).

2. Dispenser according to claim 1, **characterized in that** the projection (6) is an angled element.

3. Dispenser according to claim 1 or 2, **characterized in that** the projection (6) is formed from metal.

4. Dispenser according to at least one of the preceding claims, **characterized in that** the projection (6) is a flat profile in the form of a plain roofing tile, wherein that edge of the projection (6) which is directed towards the swab strip (2) is half-round.

5. Dispenser according to at least one of the preceding claims, **characterized in that** the swabs (1, 1') are formed from a plurality of layers, wherein the outer layers are formed from cellulose tissue and at least one inner layer of the swabs (1, 1') is formed from cellulose wadding.

6. Dispenser according to claim 5, **characterized in that** the dispenser (10, 11, 12, 14) has provided on it two projections (6) for engagement through two perforations (3) which are provided between two adjacent swabs (1, 1') and two further swabs (1, 1') which follow in the removal direction (E).

7. Dispenser according to at least one of the preceding claims, **characterized in that** the dispenser (10) is designed as a dispensing stand (7) for accommodating a swab pack (4), wherein the dispensing stand (7) has the projection (6).

8. Dispenser according to claim 7, **characterized in that**, on the dispensing stand (7), it is possible for the position of the projection (6) to be adapted to the position of the removal opening (5, 5') of the swab pack (4).

9. Dispenser according to at least one of claims 1 to 6, **characterized in that** the dispenser (11, 12) is designed as a dispensing pack (8, 8'), wherein the dispensing pack (8, 8') has the projection (6).

10. Dispenser according to at least one of the preceding claims, **characterized in that** the projection (6) is spaced apart from the guide (9).

11. Dispenser according to at least one of the preceding claims, **characterized in that** the dispenser (14), on the side on which the removal opening (5, 5') is provided, has a longer leg (15) than on the opposite side, and therefore the dispenser (14) can be set up in a tilted state.

12. Dispenser according to claim 11, **characterized in that** the leg (15) is height-adjustable.

13. Dispenser according to claim 12, **characterized in that** the leg (15) is retained laterally in sliding guides (16).

14. Dispenser according to claim 11, 12 or 13, **characterized in that** the tilting height can be defined by means of at least one arresting device provided on the leg (15).

## Revendications

1. Distributeur (10, 11, 12, 14) comprenant une bande de compresses (2) s'étendant dans une direction longitudinale (L), avec des compresses rectangulaires (1, 1') qui constituent des portions de la bande de compresses (2), la bande de compresses (2) présentant, dans une direction transversale (Q) de la bande de compresses (2), des perforations (3) au niveau desquelles les compresses (1, 1') peuvent être séparées de la bande de compresses (2), une extrémité de la bande de compresses (2) pouvant être guidée à travers au moins une ouverture de prélèvement (5, 5') du distributeur (10, 11, 12, 14) et pouvant être tirée dans une direction de prélèvement (E) hors du distributeur (10, 11, 12, 14), et la direction de prélèvement (E) étant la même que la direction longitudinale (L) de la bande de compresses (2),
**caractérisé en ce**
**qu'**au moins un rebord (6) disposé au niveau de l'ouverture de prélèvement (5, 5') et/ou après l'ouverture de prélèvement (5, 5') dans la direction de prélèvement (E) est prévu pour s'engager à travers au moins l'une des perforations (3) de la bande de compresses (2), les perforations (3) étant des fentes oblongues qui sont réalisées dans le matériau de la bande de compresses (2) et qui sont interrompues par de petites nervures, les nervures retenant ensemble les compresses (1) individuelles et le rebord (6) étant guidé à travers au moins une ouverture formée par une perforation dans la bande de compresses (2) et le rebord (6) se distinguant clairement de dents d'arrachage utilisées pour déchirer des compresses et par contre ayant une taille telle qu'il puisse s'engager à travers les perforations (3) réalisées dans la bande de compresses (2), de telle sorte que lorsque le rebord (6) se trouve dans une perforation (3), une compresse (1, 1') puisse être séparée de la bande de compresses (2), en tirant la bande de compresses (2) dans le sens de la direction de prélèvement (E) et/ou vers le bas, le rebord (6) étant donc orienté essentiellement dans la direction de la perpendiculaire à la surface de la bande de compresses (2) et le rebord (6) opposant à la bande de compresses (2) une résistance lors de la traction de la bande de compresses (2) dans la direction de prélèvement (E), de telle sorte que la bande de compresses (2) soit retenue par le rebord (6), tandis qu'une compresse (1, 1') est déchirée de la bande de compresses (2), et le distributeur (10, 12) présentant un guide (9) pour la bande de compresses (2), le guide (9) étant prévu dans la direction de prélèvement (E) de la bande de compresses (2) entre l'ouverture de prélèvement (5, 5') et le rebord (6).

2. Distributeur selon la revendication 1, **caractérisé en ce que** le rebord (6) est un coude.

3. Distributeur selon la revendication 1 ou 2, **caractérisé en ce que** le rebord (6) est réalisé en métal.

4. Distributeur selon au moins l'une quelconque des revendications précédentes, **caractérisé en ce que** le rebord (6) est un profilé plat en forme de tuile de toiture du type queue de castor, l'arête du rebord (6) tournée vers la bande de compresses (2) étant réalisée sous forme semi-circulaire.

5. Distributeur selon au moins l'une quelconque des revendications précédentes, **caractérisé en ce que** les compresses (1, 1') sont réalisées à partir de plusieurs couches, les couches extérieures étant réalisées en tissu de cellulose et au moins une couche intérieure des compresses (1, 1') étant réalisée en ouate de cellulose.

6. Distributeur selon la revendication 5, **caractérisé en ce que** deux rebords (6) sont prévus sur le distributeur (10, 11, 12, 14) pour s'engager à travers deux perforations (3) qui sont prévues entre deux compresses (1, 1') disposées l'une à côté de l'autre et deux autres compresses successives (1, 1') dans la direction de prélèvement (E).

7. Distributeur selon au moins l'une quelconque des revendications précédentes, **caractérisé en ce que** le distributeur (10) est réalisé sous forme de support de distribution (7) pour recevoir un paquet de compresses (4), le support de distribution (7) présentant le rebord (6).

8. Distributeur selon la revendication 7, **caractérisé en ce que** la position du rebord (6) peut être adaptée à la position de l'ouverture de prélèvement (5, 5') du paquet de compresses (4) au niveau du support de distribution (7).

9. Distributeur selon au moins l'une quelconque des revendications 1 à 6, **caractérisé en ce que** le distributeur (11, 12) est réalisé sous forme de paquet de distribution (8, 8'), le paquet de distribution (8, 8') présentant le rebord (6).

10. Distributeur selon au moins l'une quelconque des revendications précédentes, **caractérisé en ce que** le rebord (6) est espacé du guide (9).

11. Distributeur selon l'une quelconque des revendications précédentes, **caractérisé en ce que** le distributeur (14), du côté où se trouve l'ouverture de prélèvement (5, 5'), présente une base (15) plus longue que sur le côté opposé, de sorte que le distributeur (14) puisse être posé en position inclinée.

12. Distributeur selon la revendication 11, **caractérisé en ce que** la base (15) est réglable en hauteur.

13. Distributeur selon la revendication 12, **caractérisé en ce que** la base (15) est maintenue latéralement dans des guides à coulisse (16).

14. Distributeur selon la revendication 11, 12 ou 13, **caractérisé en ce que** la hauteur de l'inclinaison peut être fixée au moyen d'au moins un dispositif de blocage prévu sur la base (15).
